# EUROPÄISCHE PATENTSCHRIFT

(11) **EP 0 574 904 B1**
(45) Veröffentlichungstag und Bekanntmachung des Hinweises auf die Patenterteilung: **02.04.1997**
(21) Anmeldenummer: 93109648.1
(22) Anmeldetag: 16.06.1993
(51) Int. Cl.: A61N 1/05

(54) **Elektrodenvorrichtung**
Electrode device
Dispositif d'électrodes

(30) Priorität: 16.06.1992 SE 9201846
(43) Veröffentlichungstag der Anmeldung: 22.12.1993
(73) Patentinhaber: Pacesetter AB, 171 95 Solna (SE)
(72) Erfinder: Lindegren, Ulf, S-122 35 Enskede (SE); Nyman, Per, S-182 64 Djursholm (SE)
(74) Vertreter: Lettström, Richard Wilhelm

(56) Entgegenhaltungen:
- EP-A- 0 450 181
- DE-A- 2 516 848
- US-A- 4 677 990
- US-A- 4 920 980
- US-A- 4 957 110

## Beschreibung

Die Erfindung betrifft eine Elektrodenvorrichtung zur intrakorporalen Stimulation von Körpergeweben, insbesondere zur intrakardialen Stimulation von Herzgeweben mit einem Elektrodenkabel, das einen wendelförmigen, flexiblen, isolierten ersten Draht umfasst, der sich über mindestens einen wesentlichen Teil der Länge des Elektrodenkabels erstreckt und dessen Innenseite einen Kanal bildet und mit einem wenigstens teilweise leitenden Elektrodenkopf, der am distalen Ende des Elektrodenkabels angebracht ist, sowie mit einem zweiten Draht, der koaxial im Kanal angebracht ist, über die gesamte Länge des Elektrodenkabels verläuft und am distalen Ende oder im Bereich des distalen Endes des Elektrodenkabels befestigt ist.

Es ist von grosser Bedeutung, dass das Elektrodenkabel bei einer derartigen Elektrodenvorrichtung ausreichend biegsam ist, damit es bei einer Einführung in das Herz eines Patienten über eine Vene dem Weg der Vene folgen kann, ohne die Venenwände zu beschädigen. Die Einführung des Elektrodenkabels erfolgt mit Hilfe eines Mandrins, der in den Kanal des Elektrodenkabels eingeführt wird. Der Mandrin besteht aus einem Material, das ausreichend steif ist, damit das Elektrodenkabel in einer Vene verschoben werden kann. Die Steifigkeit des Elektrodenkabels kann, abhängig vom Durchmesser und Material des Mandrins, variieren. In schwierigen Passagen, in denen das Elektrodenkabel stark gebogen werden muss, wird der Mandrin häufig etwas nach hinten verschoben, so dass das distale Ende des Elektrodenkabels maximal biegsam wird. Nachdem das vordere Ende des Elektrodenkabels eine solche Stelle passiert hat, wird der Mandrin wieder an das distale Ende vorgeschoben, damit dieses Ende zum Vorhof oder zur Kammer des Herzens transportiert werden kann, bis der Elektrodenkopf gegen die Herzwand zur Stimulation anliegt. Es ist daher für den Arzt nicht ganz einfach, ein Elektrodenkabel mit Hilfe eines Mandrins durch eine Vene zu transportieren.

Eine Elektrodenvorrichtung der eingangs genannten Art ist durch die US-PS 4 677 990 bekannt. Das Ende des zweiten Drahtes ist am Elektrodenkopf befestigt und läuft teils durch den Kanal und teils exzentrisch zwischen dem ersten Draht und der äusseren Isolierung des Elektrodenkabels. Beim Ziehen des Drahtes wird das distale Ende des Elektrodenkabels derart gebogen, dass dieses Ende eine J-Form bildet. Die Einführung des Elektrodenkabels in das Herz eines Patienten erfolgt mit Hilfe eines Mandrins.

Eine weitere ähnliche Elektrodenvorrichtung ist in der DE-OS 25 16 848 beschrieben. Bei dieser Elektrodenvorrichtung hat der zweite Draht die Aufgabe, einen federbelasteten Anker, der am Elektrodenkopf angebracht ist, auszulösen. Das proximale Ende des Drahtes läuft ausserdem durch ein am proximalen Ende des Elektrodenkabels vorhandenes seitlich angeordnetes Loch. Aufgrund des Verlaufes des Drahtes durch das Elektrodenkabel wird beim Ziehen des Drahtes das Elektrodenkabel gebogen, bis das distale Ende des Elektrodenkabels eine J-Form aufweist. In dieser Schrift ist nicht beschrieben, wie die Einführung des Elektrodenkabels in das Herz eines Patienten erfolgt.

Der Erfindung liegt die Aufgabe zugrunde, eine Elektrodenvorrichtung der eingangs genannten Art zu schaffen, bei der das Elektrodenkabel mit einfachen Mitteln ohne Hilfe eines Mandrins in einer gewünschten Weise von einem extrem weichen und flexiblen in einen steifen Zustand überführt werden kann.

Diese Aufgabe ist erfindungsgemäss dadurch gelöst, dass der zweite Draht koaxial über die ganze Länge des Elektrodenkabels koaxial verläuft und sein proximales Ende ausserhalb des proximalen Endes des Elektrodenkabels liegt, wobei die Elektrodenvorrichtung Mittel aufweist, die den zweiten Draht gegenüber dem Elektrodenkabel in einer definierten Weise verschieben können. Indem mit Hilfe der Mittel der zweite Draht gestreckt wird gleichzeitig damit, dass das proximale Ende des Elektrodenkabels festgehalten wird, wird der erste wendelförmige Draht in seiner Längsrichtung zusammengedrückt. Das Elektrodenkabel wird nun, abhängig vom Zug, steifer bzw. biegsamer. Auf diese Weise kann während des Einführens die Elastizität des Elektrodenkabels von ausserordentlich weichem bis zu einem steifen Zustand verändert werden. Es ist daher nicht notwendig, bei einer Einführung eines Elektrodenkabels einen Mandrin zu verwenden.

In einer vorteilhaften Weiterbildung der Erfindung wird vorgeschlagen, dass der zweite Draht ein Leiter ist, der mit dem Elektrodenkopf verbunden ist. Der Leiter kann nun sowohl den zweiten Draht als auch einen üblicherweise wendelförmigen Leiter, der mit dem Elektrodenkopf verbunden ist, ersetzen, was zu einem einfacheren Aufbau der Elektrodenvorrichtung führt.

Eine günstige Ausgestaltung der Erfindung ergibt sich, wenn der wendelförmige erste Draht derart verläuft, dass lediglich das distale Ende des Elektrodenkabels von diesem Draht frei ist. Hierdurch wird erreicht, dass das Ende des Elektrodenkabels, auch wenn das übrige Elektrodenkabel in eine steife Lage gebracht ist, weich sein kann, was beim Passieren eines starken Venenbogens von Vorteil sein kann.

In einer vorteilhaften Ausbildung der Erfindung wird vorgeschlagen, dass die Mittel zur Steuerung der Spannung des zweiten Drahtes zwei gegeneinander verschiebbare Halterungen umfassen, wobei die erste Halterung am proximalen Ende des Elektrodenkabels und die zweite Halterung am proximalen Ende des zweiten Drahtes befestigt ist. Durch diese Halterungen kann eine definierte Verschiebung des zweiten Drahtes gegenüber dem Elektrodenkabel erreicht werden.

Eine weitere Ausgestaltung der Erfindung ergibt sich, in der die beiden Halterungen ineinander einschiebbare Teile bilden, wobei die zweite Halterung mit einer Schraube versehen ist, deren eines Ende gegen die freie Stirnseite der ersten Halterung anliegt, und wobei die zweite Halterung gegenüber der ersten Halterung mit Hilfe der Schraube derart verschiebar ist, dass, wenn die Schraube in die eine Richtung gedreht wird, der zweite Draht gespannt wird und wenn die Schraube in die andere Richtung gedreht wird, der Draht entspannt wird. Hierdurch kann eine weitere Verfeinerung der erwähnten Verschiebung zwischen Draht und Elektrodenkabel erreicht werden. Durch diesen Aufbau kann auch der Arzt während der Einführung sehr rasch zwischen verschiedenen Steifigkeiten des Elektrodenkabels wechseln.

Weitere Vorteile und Einzelheiten ergeben sich aus den Unteransprüchen.

Die Erfindung ist nachfolgend anhand eines in den Zeichnungen dargestellten Ausführungsbeispiels näher erläutert. Es zeigen:
- FIG 1: eine Elektrodenvorrichtung nach der Erfindung mit einem Elektrodenkabel und mit einer Anordnung im Längsschnitt, mit der ein Draht, der im Elektrodenkabel verläuft, gespannt bzw. entspannt werden kann, wobei der Draht in dieser FIG in einem entspannten Zustand gezeigt ist und
- FIG 2: eine Elektrodenvorrichtung mit einem Elektroden kabel und mit einer Anordnung nach FIG 1, bei der der Draht in einem gespannten Zustand gezeigt ist.

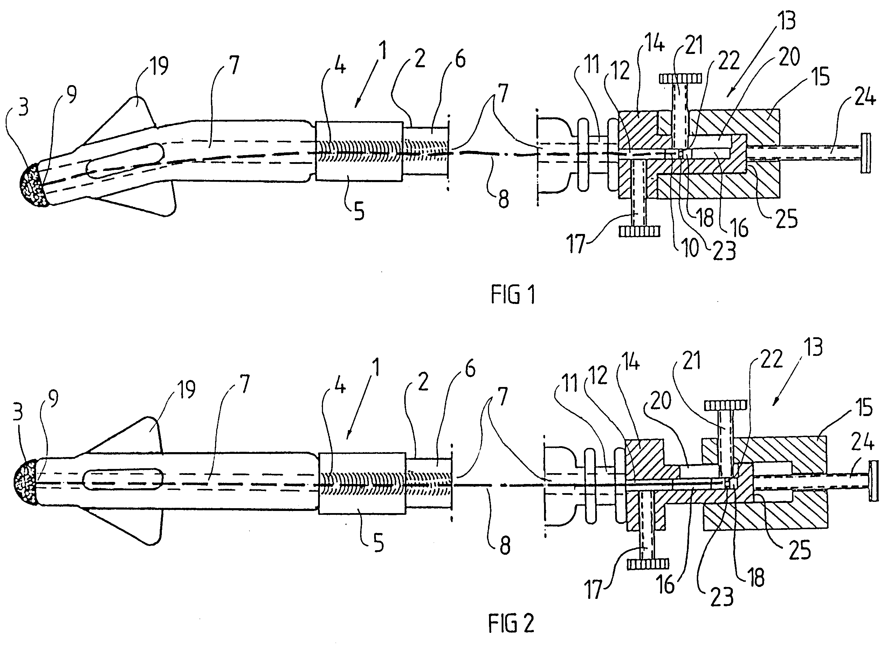

In der FIG 1 ist eine Elektrodenvorrichtung 1 zur intrakardialen Stimulation des Herzens abgebildet. Die Elektrodenvorrichtung 1, die in diesem Beispiel bipolar ist, besteht aus einem Elektrodenkabel 2, an dessen distalem Ende ein Elektrodenkopf 3 zur Stimulation von Herzgeweben eines Patienten angebracht ist. Das Elektrodenkabel 2 umfasst einen wendelförmigen, flexiblen Leiter 4, der sich vom proximalen Ende 11 des Elektrodenkabels 2 bis zu einem an dem Elektrodenkabel 2 und in der Nähe des Elektrodenkopfes 3 angebrachten Elektrodenring 5 erstreckt. Die Aussenseite des wendelförmigen Leiters 4 ist mit einer Isolierschicht 6 versehen und dessen Innenseite bildet ein Kanal 7. In diesem Ausführungsbeispiel ist ausserdem ein Kanal zwischen dem Elektrodenring 5 und dem Elektrodenkopf 3 angeordnet. Auch dieser Kanal ist mit dem Bezugszeichen 7 versehen. Gleich hinter dem Elektrodenkopf 3 sind Mittel 19 zum Fixieren des Kopfes 3 im Herzen vorgesehen. In dem Kanal 7 verläuft ein leitender Draht 8, dessen eines Ende 9 am Elektrodenkopf 3 befestigt ist und dessen anderes Ende 10 ausserhalb des proximalen Endes 11 des Elektrodenkabels 2 liegt. Der Draht 8, der koaxial über die gesamte Länge des Elektrodenkabels 2 verläuft, ist dazu vorgesehen, dem Elektrodenkopf 3 elektrische Impulse von einem Herzstimulationsgerät zur Stimulation des Herzens zuzuführen. Das distale Ende des Elektrodenkabels 2 kann, wie in der FIG dargestellt ist, vorzugsweise vorgeformt gebogen sein.

Das proximale Ende 11 des Elektrodenkabels 2 ist mit einem Stift 12 versehen, der an einem nicht dargestellten Herzstimulationsgerät angeschlossen wird, wenn die Elektrodenvorrichtung im Herzen des Patienten appliziert worden ist. Am Stift 12 ist eine Anordnung 13 angebracht, die zwei gegeneinander verschiebbare Halterungen 14, 15 umfasst, die ineinander einschiebbare Teile bilden. Die Halterung 14 ist mit einer koaxial angeordneten Bohrung 16 versehen, die etwa über die gesamte Länge der Halterung 14 verläuft. Die Bohrung 16 wird auf den Stift 12 geschoben, wobei die Halterung 14 mittels einer Schraube 17 an dem Stift 12 befestigt wird. In der Bohrung 16 ist auch eine darin verschiebbare Hülse 18 angeordnet, an der das proximale Ende 10 des Drahtes 8 fest angebracht ist. Die Hülse 18 kann vorzugsweise ein vom Stift 12 getrenntes Teil sein. Die Halterung 14 ist auch mit einem Schlitz 20 versehen, der mit der Bohrung 16 zusammenfällt und mit dieser parallel verläuft. Die zweite Halterung 15, die an der Halterung 14 verschiebbar angebracht ist, ist mit einer Schraube 21 versehen, die über den Schlitz 20 mit der Hülse 18 verbunden ist. Da die Hülse 18 nicht gegen die Wand der Bohrung 16 gedrückt werden darf, ist das eine Ende der Schraube 21 mit einem Zapfen 22 versehen, der in einem an der Hülse 18 angebrachten Nut 23 liegt. Die Halterung 15 ist auch mit einer Stellschraube 24 versehen, deren eines Ende gegen die freie Stirnseite 25 der Halterung 14 anliegt.

Wenn die Halterungen 14, 15 wie in der FIG 1 gezeigt ist dicht aneinander anliegen, liegt auch die Hülse 18 gegen die Stirnseite des Stiftes 12 an, wobei der Draht 8 entspannt ist. Wenn die Stellschraube 24 in die eine Richtung gedreht wird, wird die Halterung 15 im Verhältnis zur Halterung 14 verschoben, wobei die Schraube 21, die bei der beschriebenen Verschiebung im Schlitz 20 verläuft, gleichzeitig die in der Bohrung 16 angebrachte Hülse 18 transportiert. Hierdurch wird der Draht 8 gegenüber dem Elektrodenkabel 2 derart verschoben, dass der wendelförmige Leiter 4 zusammengedrückt wird, was die Steifigkeit des Elektrodenkabels 2 beeinflusst.

In der FIG 2 ist die äusserste ausgezogene Lage der Halterung 15 gezeigt, in der der Draht 8 maximal gespannt ist. In dieser Ausführung hat der Draht 8 auch das distale Ende des Elektrodenkabels 2 ausgerichtet. Die Stellschraube 24 kann vorzugsweise mit einer grossen Gewindesteigung versehen sein, so dass der Arzt nur mit einer kleinen Drehung der Stellschraube 24 eine sichtbare Veränderung der Steifigkeit des Elektrodenkabels 2 erhält. Mit Hilfe der Stellschraube 24, die die Spannung des Drahtes 8 steuert, kann der Arzt zwischen verschiedenen Steifigkeiten am Elektrodenkabel 2 während der Einführung wechseln. Auf diese Weise ist kein Mandrin erforderlich.

Wenn das Elektrodenkabel 2 im Herz oder in einer Vene appliziert ist, wird die Anordnung 13 vom Elektrodenkabel 2 mit Hilfe der Schrauben 17 und 21 gelöst, wobei das Elektrodenkabel 2 an ein Herzstimulationsgerät angeschlossen wird. In einer weiteren Ausführungsform der Anordnung 13 können die Schrauben 17 und 21 durch weitere Arten von Schnellverschlüssen ersetzt werden.

Ein Elektrodenkabel 2 mit einem vorgebogenen, distalen Ende kann einen Transport durch eine Venenbiegung vereinfachen. Durch Drehen des Elektrodenkabels 2 um seine Längsachse und/oder durch Ausrichten des distalen Endes mit Hilfe des Drahtes 8 kann auch eine starke Venenbiegung ohne grössere Probleme passiert werden.

Die Elektrodenvorrichtung kann nach der Erfindung auch unipolar sein. Bei einer solchen Elektrodenvorrichtung kann der wendelförmige Leiter 4 durch einen wendelförmigen Draht, der eine mechanische Stützfunktion hat, ersetzt werden, der beim Spannen des Drahtes 8 wie bereits beschrieben, derart zusammengedrückt wird, das das Elektrodenkabel steif wird.

Das Elektrodenkabel 2 der Elektrodenvorrichtung kann auch mit einem wendelförmigen Leiter 4, der mit dem Elektrodenkopf 3 verbunden ist, versehen sein. In einem solchen Fall kann der Draht 8 durch einen nichtleitenden Draht ersetzt werden.

In einem weiteren Ausführungsbeispiel einer Elektrodenvorrichtung nach der Erfindung, bei dem das Elektrodenkabel 2 mit einem Elektrodenring 5 versehen ist, der vom Elektrodenkopf 3 weiter entfernt angebracht als in der FIG 1 und 2 gezeigt ist, kann dieser Teil des Elektrodenkabels 2, der nicht mit einem wendelförmigen Leiter 4 versehen ist, mit einem wendelförmigen nichtleitenden Draht versehen sein.

### Bezugszeichenliste

- 1: Elektrodenvorrichutng
- 2: Elektrodenkabel
- 3: Elektrodenkopf
- 4: Leiter, erster Draht
- 5: Elektrodenring, leitende Oberfläche
- 6: Isolierschicht
- 7: Kanal
- 8: zweiter Draht,
- 9, 10: Drahtende, proximales Ende des zweiten Drahtes
- 11: proximales Ende des Elektrodenkabels
- 12: Stift
- 13: Anordnung, Mittel
- 14: erste Halterung
- 15: zweite Halterung
- 16: Bohrung
- 17, 21: Schraube
- 18: Hülse
- 19: Fixiermittel
- 20: Schlitz
- 22: Zapfen
- 23: Nut
- 24: Stellschraube, Schraube
- 25: Stirnseite

## Patentansprüche

1. Elektrodenvorrichtung zur intrakorporalen Stimulation von Körpergeweben, insbesondere zur intrakardialen Stimulation von Herzgeweben, mit einem Elektrodenkabel (2), das einen wendelförmigen, flexiblen, isolierten ersten Draht (4) umfasst, der sich über mindestens einen wesentlichen Teil der lange des Elektrodenkabels erstreckt und dessen Innenseite einen Kanal (7) bildet und mit einem wenigstens teilweise leitenden Elektroderkopf (3), der am distalen Ende des Elektroderkabels (2) angebracht ist, sowie mit einem zweiten Draht (8), der im Kanal (7) angebracht ist, über die gesamte Länge des Elektrodenkabels (2) verläuft, am distalen Ende (9) oder im Bereicht des distalen Endes des Elektrodenkabels (2) befestigt ist und sein proximales Ende (10) ausserhalb des proximalen Endes (11) des Elektrodenkabels (2) liegt, dadurch **gekennzeichnet**, dass der zweite Draht (8) im Kanal (7) koaxial über die ganze Länge des Elektrodenkabels (2) verlauft, und dass der Elektrodenvorrichtung Mittel (13) zugeordnet sind, die den zweiten Draht (8) gegenüber dem Elektrodenkabel (2) in einer definierten Weise verschieben können, um den wendelförmigen, flexiblen ersten Draht (8) derart zusammenzudrücken, dass die Elektrodenvorrichtung (1) von einen, weichen in einen steifen Zustand überführt werden kann.

2. Elektrodenvorrichtung nach Anspruch 1, **dadurch gekennzeichnet**, dass der zweite Draht (8) ein Leiter ist, der mit dem Elektrodenkopf (3) verbunden ist.

3. Elektrodenvorrichtung nach Anspruch 1 oder 2, **dadurch gekennzeichnet**, dass der wendelförmige erste Draht (4) derart verläuft, dass lediglich das distale Ende des Elektrodenkabels (2) von diesem Draht (4) frei ist.

4. Elektrodenvorrichtung nach einem der Ansprüche 1 bis 3, **dadurch gekennzeichnet**, dass der wendelförmige erste Draht (4) leitend und an eine leitende Oberfläche (5) am Elektrodenkabel (2) angeschlossen ist.

5. Elektrodenvorrichtuug nach Anspruch 1, **dadurch gekennzeichnet,** dass der wendelförmige erste Draht (4) leitend und an den Elektrodenkopf (3) angeschlossen ist.

6. Elektrodenvorrichtung nach einem der Ansprüche 1 bis 5, **dadurch gekennzeichnet**, dass die Mittel (13) zur Steuerung der Spannung des zweiten Drahtes (8) zwei gegeneinander verschiebbare Halterungen (14, 15) umfassen, wobei die erste Halterung (14) am proximalen Ende (11) des Elektrodenkabels (2) und die zweite Halterung (15) am proximalen Ende (10) des zweiten Drahtes (8) befestigt ist.

7. Elektrodenvorrichtung nach Anspruch 6, **dadurch gekennzeichnet**, dass die beiden Halterungen (14, 15) ineinander einschiebbbare Teile bilden, wobei die zweite Halterung (15) mit einer Schraube (24) versehen ist, deren eines Ende gegen die freie Stirnseite (25) der ersten Halterung (14) anliegt, und wobei die zweite Halterung (15) gegenüber der ersten Halterung (14) mit Hilfe der Schraube (24) derart verschiebar ist, dass, wenn die Schraube (24) in die eine Richtung gedreht wird der zweite Draht (8) gespannt wird, und wenn die Schraube (24) in die andere Richtung gedreht wird, der Draht (8) entspannt wird.

## Claims

1. Electrode device for intracorporeal stimulation of body tissues, in particular for intracardiac stimulation of heart tissues, having an electrode cable (2), which comprises a helical, flexible, insulated first wire (4), which extends over at least a considerable portion of the length of the electrode cable and the inside of which forms a channel (7), and having an electrode head (3), which is at least partially conductive and is attached to the distal end of the electrode cable (2), and also having a second wire (8), which is attached in the channel (7), extends over the whole length of the electrode cable (2), is secured to the distal end (9) or in the region of the distal end of the electrode cable (2), and the proximal end (10) of which lies beyond the proximal end (11) of the electrode cable (2), characterised in that the second wire (8) extends in the channel (7) coaxially over the whole length of the electrode cable (2), and in that means (13) are allocated to the electrode device, which means can shift the second wire (8) with respect to the electrode cable (2) in a defined manner in order to compress the helical, flexible first wire (8) (sic) in such a way that the electrode device (1) can be converted from a soft state into a rigid state.

2. Electrode device according to claim 1, characterised in that the second wire (8) is a conductor which is connected to the electrode head (3).

3. Electrode device according to claim 1 or 2, characterised in that the helical first wire (4) extends in such a way that only the distal end of the electrode cable (2) is free from this wire (4).

4. Electrode device according to one of the claims 1 to 3, characterised in that the helical first wire (4) is conductive and is connected to a conductive surface (5) at the electrode cable (2).

5. Electrode device according to claim 1, characterised in that the helical first wire (4) is conductive and is connected to the electrode head (3).

6. Electrode device according to one of the claims 1 to 5, characterised in that the means (13) for controlling the tension of the second wire (8) comprise two holding elements (14, 15) which can be shifted with respect to each other, with the first holding element (14) being secured to the proximal end (11) of the electrode cable (2) and the second holding element (15) being secured to the proximal end (10) of the second wire (8).

7. Electrode device according to claim 6, characterised in that the two holding elements (14, 15) form portions which can be pushed into each other, with the second holding element (15) being provided with a screw (24), the one end of which rests against the free end (25) of the first holding element (14), and with the second holding element (15) being able to be shifted with respect to the first holding element (14) with the aid of the screw (24) in such a way that if the screw (24) is rotated in the one direction, the second wire (8) is tensed, and if the screw (24) is rotated in the other direction, the wire (8) is released.

## Revendications

1. Dispositif à électrode pour la stimulation intracorporelle de tissus corporels, notamment pour la stimulation intracardiaque de tissus du coeur, comportant un câble (2) d'électrode, qui comprend un premier fil (4) métallique isolé souple hélicoïdal, qui s'étend sur au moins une majeure partie de la longueur du câble d'électrode et dont le côté intérieur forme un canal (7), et au moins une tête (3) d'électrode au moins partiellement conductrice, qui est montée à l'extrémité distale du câble (2) d'électrode, ainsi qu'un second fil métallique (8), qui est monté dans le canal (7), qui s'étend sur toute la longueur du câble (2) d'électrode, qui est fixé à l'extrémité (9) distale ou dans la zone de l'extrémité distale du câble (2) d'électrode et dont l'extrémité proximale (10) se trouve à l'extérieur de l'extrémité proximale (11) du câble (2) d'électrode, caractérisé en ce que le second fil métallique (8) s'étend dans le canal (7) coaxialement sur toute la longueur du câble (2) d'électrode et il est associé au dispositif à électrode des moyens (13), qui peuvent déplacer le second fil métallique (8) par rapport au câble (2) d'électrode d'une manière définie pour comprimer le premier fil métallique (8) souple hélicoïdal de telle sorte que le dispositif (1) à électrode puisse passer d'un état mou à un état rigide.

2. Dispositif à électrode suivant la revendication 1, caractérisé en ce que le second fil métallique (8) est un conducteur qui est relié à la tête (3) d'électrode.

3. Dispositif à électrode suivant la revendication 1 ou 2, caractérisé en ce que le premier fil (4) métallique s'étend de telle sorte qu'exclusivement l'extrémité distale du câble (2) d'électrode soit dégagée de ce fil métallique (4).

4. Dispositif à électrode suivant l'une des revendications 1 à 3, caractérisé en ce que le premier fil métallique (4) hélicoïdal est conducteur et est connecté à une surface conductrice (5) du câble (2) d'électrode.

5. Dispositif à électrode suivant la revendication 1, caractérisé en ce que le premier fil métallique (4) hélicoïdal est conducteur et est connecté à la tête (3) d'électrode.

6. Dispositif à électrode suivant l'une des revendications 1 à 5, caractérisé en ce que les moyens (13) de commande de la tension du second fil métallique (8) comprennent deux fixations (14, 15) pouvant être déplacées l'une par rapport à l'autre, la première fixation (14) étant fixée à l'extrémité proximale (11) du câble (2) d'électrode et la seconde fixation (15) étant fixée à l'extrémité proximale (10) du second fil métallique (8).

7. Dispositif à électrode suivant la revendication 6, caractérisé en ce que les deux fixations (14, 15) forment des pièces enfichables l'une dans l'autre, la seconde fixation (15) étant munie d'une vis (24), dont une extrémité s'applique au côté frontal libre (25) de la première fixation (14), et la seconde fixation (15) pouvant être déplacée par rapport à la première fixation (14) à l'aide de la vis (24), de telle sorte que, lorsque la vis (24) est tournée dans un sens, le second fil métallique (8) soit tendu et lorsque la vis (24) est tournée dans l'autre sens, le fil métallique (8) soit détendu.
